**Europäisches Patentamt**

(19) **European Patent Office**

**Office européen des brevets**

(11) Publication number : **0 397 273 B1**

(12) **EUROPEAN PATENT SPECIFICATION**

(45) Date of publication of patent specification :
30.03.94 Bulletin 94/13

(51) Int. Cl.⁵ : **C07C 2/12**

(21) Application number : **90201173.3**

(22) Date of filing : **09.05.90**

(54) **Process for oligomerizing olefins in order to produce liquid C5 + hydrocarbons.**

(30) Priority : **11.05.89 IT 2044389**

(43) Date of publication of application :
**14.11.90 Bulletin 90/46**

(45) Publication of the grant of the patent :
**30.03.94 Bulletin 94/13**

(84) Designated Contracting States :
**AT BE CH DE DK ES FR GB GR LI LU NL SE**

(56) References cited :
**EP-A- 0 126 527**
**US-A- 4 899 015**

(73) Proprietor : **ENIRICERCHE S.p.A.**
**Corso Venezia 16**
**I-20121 Milan (IT)**

(72) Inventor : **Soave, Giorgio**
**Via Europa 7**
**I-20097 San Donato Milanese, Milan (IT)**

(74) Representative : **De Carli, Erberto et al**
**ING. BARZANO & ZANARDO MILANO S.p.A.**
**Via Borgonuovo, 10**
**I-20121 Milano (IT)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for oligomerizing light olefins in order to produce liquid $C_5+$ hydrocarbons.

On this subject, a large number of patents exist.

Mobil Oil Corporation is one of the most active companies in this field, and is the owner of several patents relating, among others, to the use of synthetic zeolites of ZSM-5 type in order to convert light olefins with higher liquid $C_5+$ olefins being produced, and to the fractionation of the reaction products.

In US-4,456,779 to said Company, a typical process scheme is disclosed, which essentially comprises:

- adding to a liquid olefinic stream fed to the process and containing a substantial fraction of light olefins, a liquid stream of light alkanes (above all, $C_3$-$C_4$);
- pre-heating the so formed stream, containing olefins and alkanes, up to a temperature of at least 230°C;
- reacting said pre-heated stream by means of a zeolitic catalyst of ZSM-5 type, with the olefinic fraction being converted into $C_5+$ hydrocarbons in a plurality of reactors, with the reaction product being cooled at the outlet from each one of said reactors;
- removing $C_4$ hydrocarbons from the cooled reaction product, with liquid $C_5+$ hydrocarbons, which are the desired product, being separated from light alkanes, which are added to the olefinic liquid stream fed to the process.

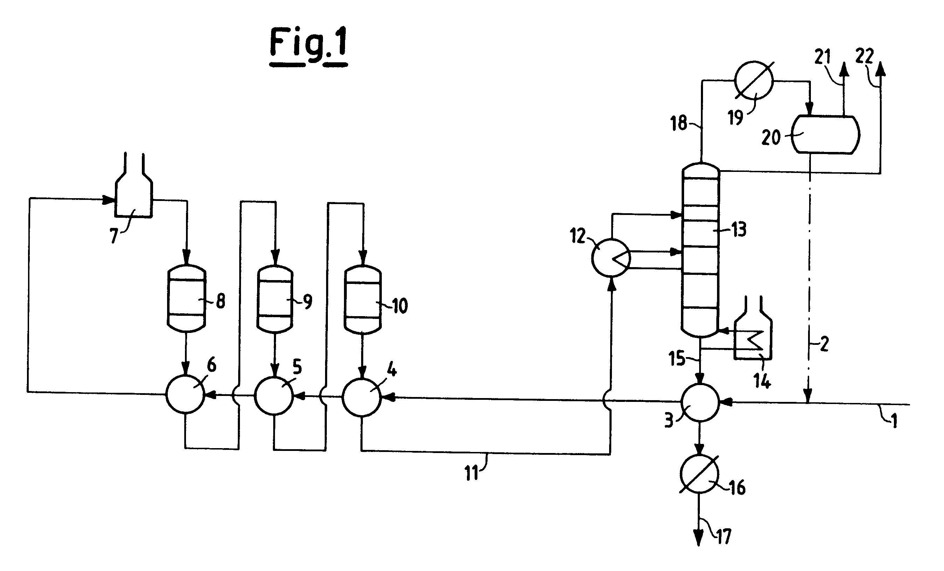

The process of US-4,456,779, which, among the processes known from the prior art, is the one which is the most similar to the process claimed by the instant Applicant in the present patent application, is described now in greater detail with the aid of the scheme shown in Figure 1.

The stream fed to the process (1), containing light olefins, after being combined with a stream (2) containing ($C_3$-$C_4$)-alkanes is pre-heated in the heat exchangers (3), (4), (5), (6) and in the furnace (7).

The preheated stream is oligomerized in the reactors in cascade (8), (9) and (10), intercalated by cooling steps respectively carried out with the heat exchangers (6), (5) and (4).

The cooled stream (11) exiting the last reactor, after being further cooled in the heat exchanger (12), is fed to the debutanizer (13), which operates under a lower pressure than of the reactors, and is also equipped, besides the reboiler (14), with the intermediate reboiler (12).

From the bottom of the tower (13) a liquid stream (15) is obtained which contains the desired product ($C_5+$ hydrocarbons), which is cooled in the heat exchangers (3) and (16) before leaving the facility (17).

From tower head (13) a gas stream (18) is obtained, which is condensed in (19) and is separated in (20) into an LPG-containing gas (21) and/or liquid (22) stream, and a liquid stream (2) above all containing ($C_3$-$C_4$)-alkanes which is recycled by being added to the feed stream (1), obviously after being pumped.

As one can observe from Figure 1, the subdivision of the reaction into a plurality of bodies is dictated by a double need:

- the reaction heat has to be removed by means of intermediate cooling steps;
- the catalyst has to be periodically regenerated.

Even if, just to make a hypothesis, the intermediate cooling steps could be avoided, the need of catalyst regeneration would anyway impose the availability of at least two bodies, i.e., the one being on stream, and the other one under regeneration. In this case, one would have a catalyst overcapacity of 100%, so that, independently from any cooling needs, subdividing the normally on-stream catalyst into a plurality of bodies, so as to reduce the unused catalyst excess to be installed in order to meet the requirements posed by the regeneration step, is more advantageous from an economical viewpoint.

The larger the number of reaction bodies under normal on-stream conditions, the smaller the required catalyst excess. On the other hand, an excessive fractionation would causes an increase in installation costs, so that exceeding the number of three on-stream bodies, i.e., the number specified by Mobil in the process scheme of Figure 1, does not seem to be advantageous.

However, even with three reaction bodies, the temperature drop inside each body is higher than the value of 30°C which the same patent recommends not to exceed.

The solution adopted by Mobil in order to solve the problem is of diluting the reactants with a suitable amount of "spent" product, collected downstream the fractionation.

Unfortunately this solution causes an increase in the bulkiness of the whole process, owing to the following reasons:

- inasmuch as the recycle is collected in the liquid state (in order to be capable of being pumped up to the pressure of the reactors), it must be subsequently evaporated and heated up to reaction temperature, with a relevant heat consumption;
- an increase occurs in circulation flowrates, and therefore in dimensions and costs of various installed equipment pieces, in particular of the heat exchangers and of the fractionation tower.

The present Applicant has found now that if the "spent" product, i.e., the stream of $(C_3-C_4)$-alkanes obtained downstream the debutanizer, is adequately exploited, the problems arisen according to the scheme of US-4,456,779 can be considerably limited and at the same time it is possible not to exceed the temperature drop of 30°C inside the reactors.

The process according to the present invention essentially comprises the following steps:

(a) a stream fed to the process containing light olefins is pre-heated up to a temperature of at least 230°C;

(b) the pre-heated feed stream is oligomerized by means of a conversion catalyst in two or more reactors in cascade, with the reaction product being cooled at the outlet from each one of said reactors;

(c) from the reaction product from the last reactors, $C_4$ hydrocarbons are removed in a distillation tower, with the desired $C_5+$ hydrocarbons being obtained at tower bottom, and an at least partially liquid stream containing $(C_3-C_4)$-alkanes being obtained overhead.

The main features of said process consists in that at least one portion of said liquid stream containing $(C_3-C_4)$-alkanes is recycled to one or more side point(s) of each individual reactor used; inside each one of said reactors the catalyst layer is subdivided into two or more beds, with said recycle stream being injected between said beds, in order that the desired temperature profile is maintained along the concerned reactor.

Secondary characteristics of such a process are evidenced by the disclosure of the reaction scheme according to the present invention, and in the example.

The amount of liquid stream recycled to the reactors preferably is of at least 15% by weight, relatively to the weight of the stream fed to the process.

Of course, such a process is not influenced by the type of conversion catalyst used: for exemplifying purposes, a catalyst of zeolitic type, such a ZSM-5, can be used.

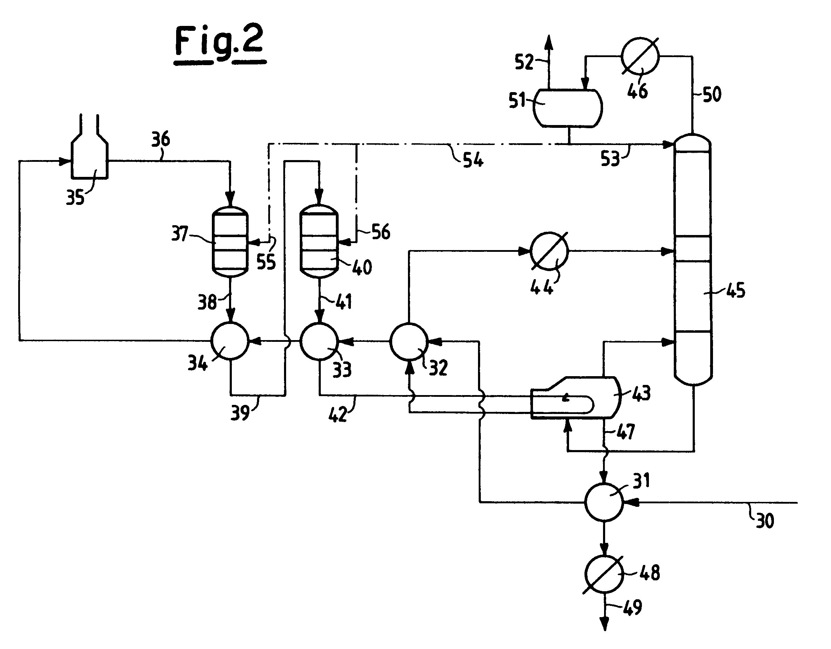

The invention is disclosed now with the aid of the scheme of Figure 2, which represents a preferred form of practical embodiment thereof.

The stream (30) fed to the process, containing light olefins, is pre-heated in the heat exchangers (31), (32), (33), (34) and in the furnace (35).

The feed stream, heated up to a temperature of at least 230°C (36) is fed to the first reactor (37) of oligomerization, from which a liquid stream (38) leaves, which is cooled in (34); the so cooled stream (39) is fed to a second reactor (40) from which a liquid stream (41) leaves, which is cooled in (33).

The cooled stream (42) is further cooled in the reboiler (43), in the heat exchanger (32) and is finally possibly adjusted at an optimal temperature in the heat exchanger (44). After being cooled in that way, the product stream is fed to the debutanizer (45) operating under a lower pressure than of the reactors, and equipped with a reboiler (43) and a condenser (46).

From the bottom of the reboiler a liquid stream (47) is collected, which contains $C_5+$ hydrocarbons, and is sent to the heat exchanger (31), wherein it is cooled, and then to the heat exchanger (48), wherein it is further cooled, before leaving (49) the facility.

From tower head (45) a gas stream (50) is obtained, which is constituted by a mixture of gases and is condensed in (46) and is separated in (51) into a gas stream (52) and a liquid stream mainly containing $(C_3-C_4)$ alkanes A portion thereof is recycled to the debutanizer as a reflux stream (53) and the remainder (54) is recycled by being injected into one or more side points of each reactor (55), (56).

An example is now given by referring to Figure 2, for the purpose of better illustrating the invention.

Example

The stream fed to the facility comes from a dehydrogenation facility, to which a stream of $(C_3-C_4)$-alkanes -- partially recycled from the oligomerization step -- is fed.

By using a ZSM-5 as the catalyst, the following conversions were obtained in the oligomerization reaction:

Ethylene     50%
Propylene   95%
Butenes     85%

and a $C_5+$ cut was produced, which was formed by:

10%   $C_5$
60%   $C_6-C_9$
30%   $C_{10}+$

The overall balance of materials of the process is reported in following Table 1.

The flowrate of the stream fed to the oligomerization is of 44,020 gh/h, with 16,464 kg/h thereof being the useful product (37.4% of fed stream; 93% of fed olefins); the balance is recycled to the de-hydrogenation.

According to the novel process scheme, according to which the stream fed to the process is not diluted, a $\Delta T$ of 30°C is obtained with approximately 1/4 of total conversion, so that the reaction was distributed over 4 beds, subdivided into 2 bodies.

The cooling between 2 beds of a same body is carried out by injecting a recycled liquid stream obtained from the overhead stream from the debutanizer, and the effluents from each body are cooled by heat exchange with other streams.

Giving the temperature a decreasing profile, with a temperature drop of 5°C less at each inlet to a new bed, the flowrates of liquid stream to be injected were of 4,632 and 4,836 kg/h; the total flowrate, i.e., 9,468 kg/h, is 21.5% of the stream fed to the facility.

The scheme of recovery adopted in this case provides for a cooling of the effluent from the first reactor, with transfer of heat to the feed stream, which is heated up to the temperature of 290°C, which is the maximum temperature which can be practically reached.

The effluent from the second reactor, leaving at 310°C, releases heat both to the feed stream and to the reboiler of the tower. In order to minimize the number of heat exchange bodies, the reboiler is installed between the two bodied of heat exchange with the feed stream.

In this way, the effluent is cooled down to 184°C, a still too high temperature, inasmuch as the computations show that the optimum result is obtained when the temperature of the stream fed to the tower does not exceed the value of 150°C. Therefore a cooler was added, which decreases the temperature of the tower feed stream to the desired temperature, dissipating heat (which possibly can be used in some other way).

Also the bottom product stream, at the temperature of 207°C, releases heat to the feed stream, with its temperature decreasing down to 135°C, and then is cooled down to storage temperature.

In this way, the only amount of live heat to be supplied is the -- very small -- amount which is required in order to increase the temperature of the feed strem from 290 up to 300°C.

A last consideration is that the tower overhead product stream (spent $C_3$ and $C_4$ alkanes) is partially obtained in the liquid state, for the injections to the reactors, and partially in gas form, to be recycled to the dehydrogenation.

A recycle in the gas state, besides reducing the amount of heat and the necessary heat exchange surface-area for the pre-heating, makes it possible the process inside the tower to be carried out under a lower pressure, and therefore with lower bottom temperatures.

## Table 1

### MATERIAL BALANCE OF OLIGOMERIZATION FACILITY

| | Oligomerization | | $C_4$ hydrocarbons removal | |
| --- | --- | --- | --- | --- |
| | Feed stream to oligomerization | Stream leaving oligomerization | Stream recycled to hydrogenation | Oligomers |
| ethane | 95.5 | 95.5 | 95.5 | 0.0 |
| ethylene | 15.6 | 7.8 | 7.8 | 0.0 |
| propane | 21,584.9 | 21,584.9 | 21,584.9 | 0.0 |
| propylene | 11,922.2 | 596.1 | 596.1 | 0.0 |
| butanes | 4,347.1 | 4,347.1 | 4,268.9 | 78.2 |
| butenes | 5,739.0 | 860.9 | 860.9 | 0.0 |
| $C_5+$ | 316.2 | 16,528.2 | 142.1 | 16,386.1 |
| | 44,020.5 | 44,020.5 | 27,556.2 | 16,464.3 |

The following Table shows further main process data.

Table 2

| | | |
|---|---|---|
| Flowrate of ($C_3$-$C_4$) hydrocarbons recycled to the reactors | kg/h | 9,468 |
| Flowrate inside the reactors | kg/h | 44,021 - 53,489 |
| Flowrate of vapours at tower head | kg/h | 75,000 |
| Pre-heating furnace | kcal/h | 285,200 |
| Head condenser | kcal/h | 3,796,400 |
| Bottom product cooler | kcal/h | 862,400 |
| Cooler of stream fed to tower | kcal/h | 1,445,000 |
| | | 6,103,800 |
| Reboiler | AxU, kcal/h/°C | 30,400 |
| Stream fed to process / 1st effluent (one body) | ditto | 36,367 |
| Stream fed to process / 2nd effluent (one body) | ditto | -- |
| Stream fed to process / last effluent (two bodies) | ditto | 64,743 |
| Stream fed to process / bottom product (one body) | ditto | 19,180 |
| | | 150,670 |

## Claims

1. Process for oligomerizing light olefins in order to produce $C_5$+ hydrocarbons, essentially comprising the following steps:

6

(a) a stream fed to the process containing light olefins is pre-heated up to a temperature of at least 230°C;

(b) the pre-heated feed stream is oligomerized by means of a conversion catalyst in two or more reactors in cascade, with the reaction product being cooled at the outlet from each one of said reactors;

(c) from the reaction product from the last reactors, $C_4$ hydrocarbons are removed in a distillation tower, with the desired $C_5+$ hydrocarbons being obtained at tower bottom, and an at least partially liquid stream containing $(C_3-C_4)$-alkanes being obtained overhead,

characterized in that at least one portion of said liquid stream containing $(C_3-C_4)$-alkanes is recycled to one or more side point(s) of each individual reactor used, wherein inside each one of said reactors the catalyst layer is subdivided into two or more beds, with said recycle stream being injected between said beds, in order that the desired temperature profile is maintained along the same reactors.

2. Process according to claim 1, wherein the amount of the liquid stream recycled to the reactors is of at least 15% by weight of the weight of the stream fed to the facility.


**Patentansprüche**

1. Verfahren zum Oligomerisieren leichter Olefine zur Ausbildung von $C_5^+$-Kohlenwasserstoffen das im wesentlichen die folgenden Stufen umfaßt:

a) ein in das Verfahren eingeführter, leichte Olefine enthaltender Strom wird auf eine Temperatur von wenigstens 230°C vorerhitzt;

b) der vorerhitzte Speisestrom wird mittels eines Umwandlungskatalysators in zwei oder mehreren, in Kaskaden angeordneten Reaktoren oligomerisiert, wobei das Reaktionsprodukt am Ausgang jedes dieser Reaktoren gekühlt wird;

c) aus dem Reaktionsprodukt des letzten Reaktors werden $C_4$-Kohlenwasserstoffe in einem Destillationsturm abgetrennt, wobei die erwünschten $C_5^+$-Kohlenwasserstoffe am Boden des Turms erhalten werden und ein wenigstens partiell flüssiger Strom mit einem Gehalt an $(C_3-C_4)$-Alkanen über Kopf erhalten wird, dadurch gekennzeichnet, daß wenigstens ein Teil des flüssigen, $(C_3-C_4)$-Alkane enthaltenden Stroms zu einem oder zu mehreren Seitenpunkten jedes einzelnen verwendeten Reaktors zurückgeführt wird, wobei innerhalb jedes dieser Reaktoren die Katalysatorschicht in zwei oder mehrere Betten unterteilt ist, wobei der Rücklaufstrom zwischen diese Betten injiziiert wird, um das gewünschte Temperaturprofil über diese Reaktoren aufrecht zu erhalten.

2. Verfahren nach Anspruch 1, worin die Menge des zu den Reaktoren zurückgeführten flüssigen Stroms wenigstens 15 Gew.-% des der Anlage zugeführten Stroms beträgt.


**Revendications**

1. Procédé d'oligomérisation d'oléfines légères, pour produire des hydrocarbures en $C_5$ et supérieurs, comportant essentiellement les étapes suivantes :

a) le courant d'alimentation du procédé, contenant des oléfines légères, est préchauffé jusqu'à une température d'au moins 230°C ;

b) le courant d'alimentation préchauffé est oligomérisé, à l'aide d'un catalyseur de conversion, dans deux réacteurs ou plus, disposés en cascade, le produit de réaction étant refroidi à la sortie de chacun de ces réacteurs ;

c) les hydrocarbures en $C_4$ sont séparés, dans une colonne de distillation, du produit de réaction sortant du dernier réacteur, les hydrocarbures en $C_5$ et supérieurs désirés étant obtenus en fond de colonne, et un courant au moins partiellement liquide et contenant des alcanes en $C_3-C_4$ étant obtenu en tête de colonne,

**caractérisé** en ce qu'au moins une partie dudit courant liquide contenant des alcanes en $C_3-C_4$ est recyclée en un ou plusieurs points latéraux de chaque réacteur utilisé, la couche de catalyseur étant divisée, dans chacun desdits réacteurs, en deux lits ou plus, et ledit courant recyclé étant injecté entre ces lits, de façon que le profil de température souhaité soit maintenu dans les réacteurs.

2. Procédé conforme à la revendication 1, dans lequel le poids du courant liquide recyclé dans les réacteurs vaut au moins 15 % du poids du courant d'alimentation de l'installation.

Fig.1

Fig.2